Europäisches Patentamt

European Patent Office

Office européen des brevets

(11) Numéro de publication: **0 447 732 A1**

# DEMANDE DE BREVET EUROPEEN

(21) Numéro de dépôt: 90480049.7

(22) Date de dépôt: **22.03.90**

(51) Int. Cl.⁵: **A61H 39/00**, A61H 39/02, A61N 1/32, A61B 5/05

Le titre de l'invention a été modifié (Directives relatives à l'examen pratiqué à l'OEB, A-III, 7.3)

(43) Date de publication de la demande: **25.09.91 Bulletin 91/39**

(84) Etats contractants désignés: **AT BE CH DE DK ES FR GB GR IT LI LU NL SE**

(71) Demandeur: **ALLOY, André Yves Jules Villa "Athéna" 190, Av. de l'Europe F-06210 Mandelieu(FR)**

Demandeur: **Feber, Alain Les jardins du Soleil, 11, rue Sidi Brahim F-06130 Grasse(FR)**

(72) Inventeur: **ALLOY, André Yves Jules Villa "Athéna" 190, Av. de l'Europe F-06210 Mandelieu(FR)**
Inventeur: **Feber, Alain Les jardins du Soleil, 11, rue Sidi Brahim F-06130 Grasse(FR)**

(74) Mandataire: **Nuss, Pierre et al 10, rue Jacques Kablé F-67000 Strasbourg(FR)**

(54) **Appareil automatique de détection et de stimulation des points d'acupuncture, avec diagnostic.**

(57) Procédé électronique entièrement automatique de stimulation des points d'acupuncture, de mésothérapie et de réflexothérapie.

L'invention concerne un procédé basé sur l'utilisation de circuits électroniques agissant par l'intermédiaire d'une électrode à pointe douce sur les points d'acupuncture, de mésothérapie et de réflexo-thérapie en déterminant exactement la position des points et les niveaux d'énergie à ramener à la normale, en calculant, puis en injectant ou en soustrayant la quantité exacte d'énergie pendant le temps voulu pour ramener l'équilibre énergétique à la normale.

EP 0 447 732 A1

Fig.1

La présente invention concerne un procédé de stimulation électrique des points chinois d'acupuncture d'auriculothérapie, de mésothérapie et de des points chinois d'acupuncture, d'auriculothérapie, de mésothérapie et de réflexothérapie entièrement automatique au moyen de circuits électroniques.

Tout le monde a, bien sûr, entendu parler de l'acupuncture, et, pour tout le monde, cette technique venue d'Extrême-Orient, apparaît comme très mystérieuse et très compliquée. Il faut savoir que l'acupuncture est d'abord le reflet de la tradition philosophique pour qui l'énergie et la matière sont une continuité. On retrouve là, de façon frappante, le système binaire d'Einstein.

Cette dualité, le Yin et le Yang du corps humain, se complète et se continue toute la vie du sujet. Il y a un écoulement continu de cette énergie vitale sur la totalité de la surface corporelle et sur des lignes privilégiées qui correspondent aux organes profonds, lignes qu'en Occident nous appelons méridiens. Si l'énergie s'arrête de s'écouler, une sorte de de noeud se forme quelque part sur le trajet de ces méridiens, la maladie apparaît. Or, sur ces trajets il y a des points, 361 en tout, plus quelques uns épars qui permettent de modifier qualitativement et quantitativement l'écoulement de l'énergie. Le diagnostic classique d'un déstquilibre de l'énergie, qualitatif et quantitatif ne peut se faire que par la prise des pouls radiaux ou "pouls chinois" ce qui nécessite une longue pratique. Une fois le déséquilibre de l'énergie bien établi et diagnostiqué, il va falloir traiter.

Pour ce faire, il faut détecter les points, et les stimuler, en posant des aiguilles. Puis il faut définir dans quel sens des aiguilles on va imprimer un mouvement de rotation aux aiguilles, soit dans le sens des aiguilles d'une montre pour "tonifier", soit dans le sens inverse pour "disperser". Tout ce processus ne pouvait être jusqu'ici que le fait des médecins spécialisés. Il existe, bien sûr, depuis longtemps des appareils dits "d' acupuncture électronique" qui permettent de détecter les points et de "traiter" en envoyant des impulsions électrostatiques. Existent également de nombreux ouvrages de vulgarisation qui permettent une localisation des points et donnent des schémas de traitement. Toutefois l'utilisateur de ces appareils se heurtait à deux obstacles majeurs: d'abord, comment décider si l'on devait tonifier ou disperser l'énergie du méridien? ensuite qúétait la forme de courant appropriée à utiliser et de quelle durée devait être le traitement?

La présente invention a permis de s'affranchir de ces difficultés d'après les études réalisées sur l'anésthésie par acupuncture qui a permis de se rendre compte des progrès possibles.

- d'abord la découverte des mécanismes de l'action d'acupuncture
- la découverte de nouveaux points d'acupuncture au niveau de l'oreille

et, surtout

1) la spécialisation de plus en plus grande des points. Il s'agit de points ayant une action constante sur un organe ou une région donnée, au nombre de deux, trois ou quatre, divisés en points principaux qui suffisent pour obtenir l'effet désiré et points secondaires plus spécialisés. C'est ce progrès considérable qui a permis de mettre la méthode pratiquement entre toutes les mains.

2) L'utilisation de nouveaux modes d'actions. On a ainsi démontré qu'on pouvait obtenir les résultats en appliquant un faible courant électrique directement sur la peau. Ceci a permis de lever un des derniers obstacles, car les résultats sont incomparablement meilleurs avec des traitements en série ; des séances de quelques minutes tous les jours, pendant des semaines, qu'avec le coup par coup.

Restait donc à lever la dernière difficulté, et non la moindre, c'est-à-dire avoir la possibilité d'appliquer le traitement exact et indiqué, sans erreur possible. Le problème était jusqu'ici insoluble. La solution, l'acupuncteur élecronique de l'Ingénieur A. FEBER et du Docteur A.ALLOY, l'apporte aujourd'hui et sur tous les plans, avec, en plus, la possibilité de stimuler les points de "mésothérapie" en effet,

selon la présente invention:

1) l'appareil détecte le point d'acupuncture et signale qu'on l'a trouvé

2) on laisse alors l'électrode en contact avec ce point; l'appareil calcule alors s'il doit disperser ou tonifier l'énergie du méridien dont dépend le point.

3) Ceci fait, il calcule la quantité de traitement à délivrer, la forme de courant et sa durée, au total en quelques fractions de seconde.

4) A la fin du traitement l'appareil signale "fin de traitement".

Donc aucune erreur possible, mais au contraire une efficacité maximum, avec une totale innocuité due à la suppression des aiguilles.

5) L'appareil est de plus utilisable sur tous les points de mésothérapie avec de remarquables résultats qui viennent compléter ceux de l'acupuncture.

Par le procédé de l'invention, nous avons donc à notre disposition une méthode souple qui permet de soulager de nombreux maux sans produits chimiques

- Qui peut être mise entre toutes les mains
- Qui peut, sans danger, être associée à toutes les thérapeutiques.

Il s'agit sans nul doute, d'une des voies d'avenir de la médecine.

Il existe d'excellents ouvrages de vulgarisation de la méthode, rédigés par des médecins acupuncteurs, qui permettent de repérer avec la plus grande facilité les points à traiter. L'appareil FEBER-ALLOY fera le reste.

Lorsqu'un organisme humain souffre d'une affection, un déséquilibre énergétique apparaît entre les deux corps médians, c'est-à-dire qu'une différence de potentiel prend naissance entre le point chinois et la partie adverse du corps, environ un microvolt à un millivolt continu, plus une résistivité variant entre 10 Kilo-Ohms à 470 Kilo-Ohms. L'expérience a montré que les valeurs comprises entre dix et cent cinquante K.O devaient être dispersées et qu'entre cent cinquante à quatre cent soixante dix K.O elles devaient être tonifiées. Seule la quantité d'énergie à injecter doit être variable.

## CARACTERISTIQUES DE L'APPAREIL

1) il localise le point à traiter par un signal sonore et lumineux continu.

2) il signale par diode lumineuse si le point à traiter va être dispersé ou tonifié.

3) il détermine la quantité d'énergie à injecter

4) si l'électrode est maintenue plus de deux secondes sur un point le traitement se déclenche et est mis en évidence par un signal sonore et lumineux hachuré à la fréquence de 16 Hertz par seconde

5) Temps de traitement de 1 à 30 secondes selon le point à traiter et arrêt signalé par signal sonore et lumineux continu, et l'on passe au point à traiter suivant.

6) Arrêt automatique de l'alimentation générale dans un délai de 60 secondes. si l'appareil n'est plus sollicité.

## REALISATION - L'ensemble est réalisé autour de quatre circuits intégrés de technologie C-MOS, permettant une très faible consommation, trois sont des porte "NAND" à raison de six portes par CI, un seul est commutateur D/A à quatre voies, monté en inverseur; un transistor est utilisé en amplificateur de courant; les composants actifs et passifs sont insérés sur un circuit inpriméen époxy.

## FONCTIONNEMENT DE L'INTERRUPTEUR AUTOMATIQUE DE L'INVENTION

La fonction Marche et Arrêt automatique est assurée par un circuit intégré composé de 6 portes "NAND" : CI 13, 14, 15, 16, 17, 18 (voir schéma en annexe).

La mise sous tension se fait par un contact fugitif avec la pastille M 1 métallique et l'électrode pointe. Une tension positive de fuite est appliquée sur l'entrée du CI 13 à travers R 18. Le 1 logique appliqué à l'entrée va fournir un zéro fugitif en sortie CI 13 déchargeant C 11 à travers D 14, l'entrée de CI 14 est à zéro, sa sortie est à 1, l'entrée de CI 15, 16, 17, 18 à 1 et la sortie de ces quatre portes en parallèles se retrouvent à zéro assurant la commutation négative U de la pile 9 Volts. Les quatre portes en parallèles assurent une faible chute de tension à la commutation. Le zéro en sortie de CI 15, 16, 17, 18 est fixé à 60 secondes par le réseau R 19 + C 11 si l'appareil n'est pas sollicité, sinon C 11 sera déchargé à travers D 12 et CI 6 dont la sortie se retrouve à zéro a chaque recherche des points chinois. Le réseau R 20 + D 13 assure un basculement franc et un verrouillage dans la phase hors tension. L'alimentation de CI 13, 14, 15, 16, 17, 18 se fait à travers R 17 limitant le courant qui serait destructeur et consommateur lors des transitions hors et sous tension ; R 16 limite le courant dans la L.E.D. 3, témoin de mise sous tension. C 25 est là pour découpler l'alimentation commutée.

## FONCTIONNEMENT DES CIRCUITS DE RECHERCHE DES POINTS D'ACUPUNCTURE ET DU SELECTEUR DE MODE (Tonification ou Dispersion)

Ces deux fonctions de l'invention sont assurées par deux circuits intégrés identiques ayant chacun 6 portes "NAND" (CI 1, 2, 3, 7, 8, 9, 10, 11, 12), un transistor NPN (TR1) et un CI à quatre voies analogiques Switch (D/A), les postes "NAND" CI 4, 5, 6 étant réservés à d'autres fonctions expliquées dans ce qui suit.

Au repos, apres la mise sous tension, la sortie de CI 7 est au niveau 1 à travers R 3 et R 12. Ce niveau est maintenu aux entrées de CI 8 et CI 1, passe a travers R 1 et D 1 vers le commutateur D/A. Si les bornes de l'inverseur B 1 et B 2 ne sont pas utilisés (pas de résistance), tous restent dans cet état. Si la résistance du point chinois est inférieure 470 K et est présente entre B 1 et B 2, l'entrée de CI 1 passe au zéro logique et sa sortie passe au 1 logique. L'avertisseur sonore Z 1 se trouve sous tension à travers R 4 et R 3 et se met à fonctionner. Le réseau R 2 + C 2 fournit un 1 logique sur l'entrée de CI 2 au bout de deux secondes. La sortie de CI 2 passe à zéro, en mettant le potentiel positif du buzzer (Z 1) à zéro à travers D 3. Par la diode D 4, CI 2 déclenche ensuite le multivibrateur composé de CI 4 et CI 5 réglé sur 16 HZ, arrête la charge du condensateur C 5 à travers la diode D 5 ; la sortie de CI 3 se trouve au 1 logique et permet à travers la diode D 6 de libérer la tension positive stockée dans C 5 et de rendre conducteur TR 1. L'émetteur de TR 1 se trouve au 1 logique, ainsi que l'entrée de CI 7 ; la sortie de CI 7 se retrouve à zéro. La tension d'émetteur va décroître à travers R 7 et D 7, la câthode de D 7 va recevoir les impulsions à travers C 3 du multivibrateur CI 4 - CI 5, la composante continue avec les impulsions superposées vont s'acheminer à l'entrée de l'inverseur D/A et vont être commutées en sortie sur la résistance du corps humain trouvée sur un point chinois à traiter. Quand la charge de C 5 sera épuisé, plus ou moins vite selon la résistance du point chinois, l'entrée de CI 7 passera à zéro et basculera sa sortie au 1 logique, retrouvant les conditions de départ c'est-à-dire un signal sonore continu plus une des deux diodes L. E. D. 1 ou L. E. D. 2 allumée indiquant que le traitement est terminé. Pendant la phase de traitement, le signal sonore et le signal lumineux sont modulés à 16 HZ par R 6 pour le buzzer Z 1 et R 10 via CI 6 pour les diodes ; R 11 sert à limiter le courant des deux diodes, L. E. D. 1 et L. E. D. 2.

Pour les points chinois présentant une résistance comprise entre 150 KOHMS et 470 KOHMS, seul le CI 1 basculera et la diode verte s'allumera. Pour des résistances de quelques OHMS à 150 KOHMS, le CI 1 et le CI 8 basculeront commutant la diode L. E. D. 1 et positionnant l'inverseur en position inverse, la commutation est assurée par CI 9 et CI 10 en parallèle avec CI 11 et CI 12, afin d'assurer le minimum de chute de tension à la commande des diodes L.E.D. 1 ou L. E. D. 2 ; R 15 + C 10 assurent le basculement et le verrouillage de CI 9 et CI 10 ; D 9 sert d'aiguilleur et maintient le choix trouvé (dispersion ou tonification) ; D 10 permet le retour à l'état initial de l'inverseur avec CI 8 à zéro en sortie ; R 14 limite le courant de commande, C 8 permet une transition franche dans les choix du traitement (dispersion ou tonification) aux valeurs proches de 150 KOHMS ; D 1 empêche la tension continue de traitement de remonter vers CI 1 ; D 8 commute les diodes L.E.D. 1 ou L.E.D. 2 à travers CI 6 en phase recherche et traitement.

Les caractéristiques des circuits électroniques de cet invention permettent de le régler sur des fréquences de quelques Hertz à 400 Hertz et de l'utiliser en stimulation pour la mésothérapie, la réflexothérapie et la thérapeutique percutanée par électro-osmose.

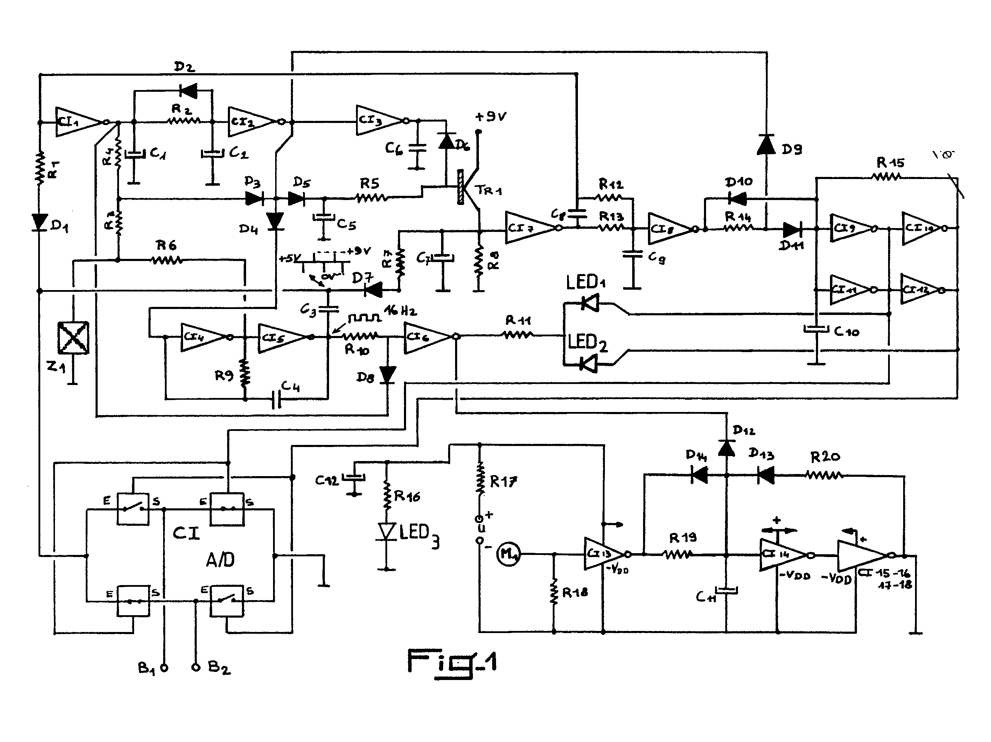

Le fonctionnement du dispositif sera mieux compris en se référant à la figure 1.

**Revendications**

1. Dispositif électronique entièrement automatique de stimulation des points d'acupuncture chez l'home et chez l'animal, caractérisé en ce qu'il comporte des circuits électroniques intervenant successivement dans un ordre prédeterminé tout en s'informant mutellement par circulation de signaux, permettant ainsi par le commutateur A/D et les portes " NAND " CI 1 et CI 8 la localisation précise des points d'acupuncture, par le commutateur A/D et les portes "NAND " CI 1, CI 8, CI 9, CI 10, CI 11, CI 12 et CI 6 la détermination du sens et du niveau du déséquilibre énergétique dans le méridien considéré, par CI 2, CI 3, CI 4, et CI 5, TR 1, D 5 et D 6, C 5 et C 6, l'application du traitement sous forme de stimulation appropriée, dont la fin est signalée à la fois par des signaux sonores et visuels. Ces mêmes circuits, avec des électrodes adaptées, permettent l'utilisation de l'appareil en mésothérapie, en réflexothérapie et en thérapie percutanée par l'électro-osmose.

2. Dispositif selon la revendication 1, caractérisé en ce qu'il comporte, pour l'application des stimulations en acupuncture et en réflexothérapie, une électrode à pointe douce, pour l'application en électro-osmose une électrode en plaque, lamellaire ou circulaire, le circuit étant fermé par une masse conductrice en contact avec le malade en un point différent du corps.

3. Dispositif électronique selon les revendications 1 et 2, caractérisé en ce qu'il comporte un commutateur A/D à quatre voies, deux portes " NANS " CI 1 et CI 8 qui permettent de déterminer avec précision les points d' acupuncture recherchés, objectivés par des signaux sonores et lumineux générés par cinq portes " NAND " CI 9, CI 10, CI 11, CI 12, et CI 6 , deux diodes LED (verte et rouge) et un buzzer électronique Z 1.

4. Le circuit électronique d'analyse du niveau d'energie dans le méridien considéré est caractérisé en ce qu'il comporte, selon les revendications 1, 2 et 3, un commutateur A/D à 4 voies, 7 portes " NAND" CI 1, CI 8, CI 9, CI 10, CI 11, CI 12 et CI 6 qui permettent de déterminer si le méridien sur lequel se trouve le point d'acupuncture considéré est surchargé ou bien présente un déficit en énergie, ce qui est mis en évidence par des signaux lumineux émis par les iodes LED verte et rouge.

5. Dispositif selon les revendications 1, 2, 3 et 4, caractérisé en ce que les circuits électroniques composés d'un commutateur A/D 4 voies et de 5 portes "NAND" CI 1, CI 8, CI 9, CI 10, CI 11, CI 12 déterminent le niveau d'énergie dans le méridien considéré, calculent par les circuits composés de 2 portes " NAND " CI 2, CI 3, du transistor TR 1, des diodes D 5, D6 et des capacités C 5, C 6, la quantité d'énergie à fournir ou à soustraire du méridien considéré.

6. Dispositif selon les revendications 1, 2, 3, 4 et 5, caractérisé en ce que les circuits électroniques composés de deux portes " NAND " CI 4, CI 5, d'un transistor TR 1, ainsi que de C 5, C 7, C 3, D 7, R 5 et le commutateur A/D envoient ou soustraient automatiquement et exactement la quantité d' énergie nécessaire pour rétablir l'équilibre énergétique normal dans le méridien considéré et signalent la fin du traitement par un signal sonore et lumineux continu.

7.  Dispositif selon les revendications 1, 2, 3, 4, 5, 6 , caractérisé en ce que les circuits électroniques composés de 6 portes "NAND " CI 13, CI 14, CI 15, CI 16, CI 17, CI 18, de D 12, de C 11 R 18, 5 19, R 20 arrêtent automatiquement l'alimentation générale de l'appareil si celui-ci reste plus de 60 secondes sans être utilisé, la mise sous tension se faisant par simple touché sur la pastille métalmise sous tension se faisant par simple touché sur la pastille métallique reliée à l'entrée de CI 13.

8.  Dispositif selon les revendications 1, 2, 3, 4, 5, 6 et 7 caractérisé en ce que les circuits électroniques définis dans ces revendications permettent, par le moyen d' électrodes à pointe mousse l'utilisation de l' appareil en acupuncture, mésothérapie et réflexothérapie, et par le moyen d' électrodes annulaires, rubannées ou en plaques l'utilisation en thérapie par électroosmose.

Fig. 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 473 882 (DELOFFRE)<br>* Page 1, lignes 1-16; page 2, lignes 2-12,28-37; page 4, ligne 13 - page 5, ligne 3; page 6, lignes 4-15; page 9, ligne 26 -page 10, ligne 20; page 15, lignes 7-29; figure 4 * | 1,3-6 | A 61 H 39/00<br>A 61 H 39/02<br>A 61 N 1/32<br>A 61 B 5/05 |
| Y |  | 2,8 |  |
| A |  | 7 |  |
| | — — — | | |
| X | FR-A-2 506 612 (RAINEAU)<br>* Page 1, lignes 1-4; page 1, ligne 24 - page 2, ligne 4; page 2, lignes 15-30; page 4, lignes 13-19; figure 2 * | 1,3,4 | |
| | — — — | | |
| X | DE-A-3 202 010 (SANDER ELEKTRONIC AG)<br>* Revendications; figures 1-2 * | 1,3-6 | |
| | — — — | | |
| X | EP-A-0 269 796 (GU)<br>* Résumé; figure 1 * | 1 | |
| A |  | 3-6 | |
| | — — — | | |
| X | FR-A-2 441 294 (BRETTE)<br>* Page 1, lignes 8-14,16-26; page 1, ligne 36 - page 2, ligne 5; revendications; figures * | 1,3-6 | |
| | — — — | | **DOMAINES TECHNIQUES RECHERCHES (Int. Cl.5)** |
| X | WO-A-8 401 516 (BEALE)<br>* Résumé; page 1, ligne 31 - page 3, ligne 14; figure 1 * | 1,2,8 | A 61 H<br>A 61 N<br>A 61 B |
| A |  | 3-6 | |
| | — — — | | |
| X | US-A-4 016 870 (LOCK)<br>* Résumé; figures 2-3 * | 1 | |
| A |  | 3-6 | |
| | — — — | | |
| Y | EP-A-0 145 176 (WACO CORP. OVERSEAS LTD)<br>* Résumé; page 9, lignes 2-11; revendications 1-3; figures 1,3,10-13 * | 2,8 | |
| | — — — | | |
| | -/- | | |

Le présent rapport de recherche a été établi pour toutes les revendications

| Lieu de la recherche | Date d'achèvement de la recherche | Examinateur |
|---|---|---|
| La Haye | 30 novembre 90 | ZEINSTRA H.S.J.H. |

CATEGORIE DES DOCUMENTS CITES
X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

& : membre de la même famille, document correspondant

**Office européen
des brevets**

# RAPPORT DE RECHERCHE
# EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. CI.5) |
|---|---|---|---|
| A | EP-A-0 029 245 (SIEMENS AG) <br> * Résumé; figures 1,3,9 * <br> — — — | 2,8 | |
| E | FR-A-2 636 525 (FEBER et al.) <br> * Le document en entier * <br> — — — — — | 1-8 | |

DOMAINES TECHNIQUES
RECHERCHES (Int. CI.5)

Le présent rapport de recherche a été établi pour toutes les revendications

| Lieu de la recherche | Date d'achèvement de la recherche | Examinateur |
|---|---|---|
| La Haye | 30 novembre 90 | ZEINSTRA H.S.J.H. |

CATEGORIE DES DOCUMENTS CITES
X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons
-------------------------------------------------------------
& : membre de la même famille, document correspondant